(19) **Europäisches Patentamt / European Patent Office / Office européen des brevets**

(11) **EP 4 620 744 A1**

(12) **EUROPEAN PATENT APPLICATION**
published in accordance with Art. 153(4) EPC

(43) Date of publication:
**24.09.2025 Bulletin 2025/39**

(21) Application number: **23903943.1**

(22) Date of filing: **12.12.2023**

(51) International Patent Classification (IPC):
**B60R 21/013** (2006.01)    **G04G 21/02** (2010.01)
**B60R 21/01** (2006.01)    **B60R 21/00** (2006.01)

(52) Cooperative Patent Classification (CPC):
**B60R 21/00; B60R 21/01; B60R 21/013;
G04G 21/02**

(86) International application number:
**PCT/KR2023/020381**

(87) International publication number:
**WO 2024/128740 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.12.2022 KR 20220173967
02.02.2023 KR 20230014309**

(71) Applicant: **Samsung Electronics Co., Ltd.
Suwon-si, Gyeonggi-do 16677 (KR)**

(72) Inventors:
• **KIM, Hyeonseong
  Suwon-si Gyeonggi-do 16677 (KR)**
• **PARK, Jeongmin
  Suwon-si Gyeonggi-do 16677 (KR)**

(74) Representative: **HGF
HGF Limited
1 City Walk
Leeds LS11 9DX (GB)**

(54) **ELECTRONIC DEVICE FOR SENSING CAR COLLISION ACCIDENT SITUATION, METHOD FOR OPERATING SAME, AND RECORDING MEDIUM**

(57)    An electronic device (201) according to an embodiment may comprise a sensor module (210), a GNSS module (240), a communication module (250), and a processor (220). The processor according to an embodiment may be configured to form first communication connection to a first external electronic device (202) worn by a user through the communication module. The processor according to an embodiment may be configured to attempt second communication connection to a second external electronic device (204) around the electronic device if the user is confirmed as aboard a car through at least one of the sensor module, the GNSS module, and the communication module. The processor according to an embodiment may be configured to transmit a trigger signal to the first external electronic device and the second external electronic device upon sensing a collision of the car through at least one of the sensor module and the GNSS module after the second communication connection is formed. The processor according to an embodiment may be configured to receive information sensed by the first external electronic device and the second external electronic device from each of the first external electronic device and the second external electronic device on the basis of transmission of the trigger signal. The processor according to an embodiment may be configured to identify information regarding the car's accident caused by the collision on the basis of the sensed information.

EP 4 620 744 A1

START

CONFIGURE FIRST COMMUNICATION
CONNECTION WITH FIRST EXTERNAL
ELECTRONIC DEVICE WORN BY USER — 301

EXECUTE ACCIDENT DETECTION APPLICATION — 303

IDENTIFY THAT USER GOT IN CAR — 305

ATTEMPT SECOND COMMUNICATION CONNECTION
WITH SECOND EXTERNAL ELECTRONIC DEVICE OF
ANOTHER PERSON AROUND ELECTRONIC DEVICE AND
CONFIGURE SECOND COMMUNICATION CONNECTION — 307

DETECT COLLISION AND TRANSMIT TRIGGER SIGNAL — 309

RECEIVE SENSING INFORMATION FROM EACH OF
FIRST EXTERNAL ELECTRONIC DEVICE AND
SECOND EXTERNAL ELECTRONIC DEVICE — 311

IDENTIFY ACCIDENT INFORMATION OF CAR,
BASED ON SENSING INFORMATION — 313

PROVIDE NOTIFICATION — 315

END

FIG. 3

## Description

[Technical Field]

Embodiments of the disclosure relate to an electronic device for detecting a car collision accident situation, a method of operating the same, and a recording medium.

[Background Art]

With the development of electronic, information, and communication technologies, various functions are being included in one electronic device. For example, a smartphone has a communication function and a sensing function of detecting surrounding situations, and more various functions may be implemented in the smartphone through additional application installation. The electronic device may not only execute an installed application or a stored file but also access a server or another electronic device in a wired or wireless manner to receive various pieces of information in real time.

As functions of the electronic device have advanced and the electronic device has become smaller, a user can use the electronic device while carrying the electronic device daily. The development of information communication technologies increases a degree of integration of the electronic device, thereby making the electronic device conveniently carried and used. The electronic device frequently used in a carried state may be exposed to various operation states. For example, the operation environment of the electronic device may vary depending on indoor/outdoor movement or a change in weather, and the electronic device may provide an optimal usage environment to the user by controlling an output device such as a display according to the operation environment.

The information may be provided as the related art to help understanding of the disclosure. Any opinion or decision on whether the above-mentioned content can be applied as the prior art related to the disclosure has been not provided.

[Detailed Description of the Invention]

[Technical Solution]

An electronic device 201 according to an embodiment may include a sensor module 210, a GNSS module 240, a communication module 250, and a processor 220. The processor according to an embodiment may be configured to establish a first communication connection with a first external electronic device 202 worn by a user through the communication module. The processor according to an embodiment may be configured to, when it is identified through at least one of the sensor module, the GNSS module, or the communication module that the user is in a car, attempt a second communication connection with a second external electronic device 204 around the electronic device. The processor according to an embodiment may be configured to, when a collision of the car is detected through at least one of the sensor module or the GNSS module after the second communication connection is established, transmit a trigger signal to the first external electronic device and the second external electronic device. The processor according to an embodiment may be configured to, based on the trigger signal being transmitted, receive information sensed by the first external electronic device and the second external electronic device from the first external electronic device and the second external electronic device respectively. The processor according to an embodiment may be configured to identify information on an accident of the car caused by the collision, based on the sensed information.

A method of operating an electronic device 201 according to an embodiment may include establishing a first communication connection with a first external electronic device 202 worn by a user. The method of operating the electronic device according to an embodiment may include, when it is identified through at least one of a sensor module 210, a GNSS module 240, or a communication module 250 included in the electronic device that the user is in a car, attempting a second communication connection with a second external electronic device 204 around the electronic device. The method of operating the electronic device according to an embodiment may include, when a collision of the car is detected through at least one of the sensor module or the GNSS module after the second communication connection is established, transmitting a trigger signal to the first external electronic device and the second external electronic device. The method of operating the electronic device according to an embodiment may include, based on the trigger signal being transmitted, receiving information sensed by the first external electronic device and the second external electronic device from the first external electronic device and the second external electronic device respectively. The method of operating the electronic device according to an embodiment may include identifying information on an accident of the car caused by the collision, based on the sensed information.

A non-transitory computer-readable recording medium 130 storing a program according to an embodiment may store a program that is able to execute establishing a first communication connection between an electronic device 201 and a first external electronic device 202 worn by a user, when it is identified through at least one of a sensor module 210, a GNSS module 240, or a communication module 250 included in the electronic device that the user is in a car, attempting a second communication connection with a second external electronic device 204 around the electronic device, when a collision of the car is detected through at least one of the sensor module or the GNSS module after the second communication connection is established, transmitting a trigger signal to the first external electronic device and the second external electronic device, receiving information sensed by the first external electronic device and the second external electronic device from the first external electronic device and the second external electronic device respectively, based on the trigger signal being transmitted, and identifying information on an accident of the car caused by the collision, based on the sensed information.

[Brief Description of Drawings]

FIG. 1 is a block diagram of an electronic device within a network environment according to various embodiments.

FIG. 2A is a diagram illustrating an electronic device and a first external electronic device of a user in a car, and a second external electronic device of a passenger according to an embodiment.

FIG. 2B is a block diagram of the electronic device, the first external electronic device, the second external electronic device, and a server according to an embodiment.

FIG. 3 is a flowchart illustrating an operation in which the electronic device identifies accident information of a car according to an embodiment.

FIG. 4 is a flowchart illustrating an operation in which the electronic device receives sensing information from the surrounding second external electronic device according to an embodiment.

FIG. 5 is a diagram illustrating an operation in which the electronic device receives sensing information from the surrounding second external electronic device according to an embodiment.

FIG. 6A is a diagram illustrating an operation in which the electronic device and the second external electronic device perform an accident detection function of the car according to an embodiment.

FIG. 6B is a diagram illustrating an operation in which the second external electronic device displays a message for sharing sensing information according to an embodiment.

FIG. 7 is a flowchart illustrating an operation in which the first external electronic device or the second external electronic device is synchronized with the electronic device, based on a trigger signal according to an embodiment.

FIG. 8A is a flowchart illustrating an operation in which the electronic device identifies an accident state, based on an acceleration value and a heart rate according to an embodiment.

FIGS. 8B and 8C are diagrams illustrating an operation in which the electronic device identifies the accident state, based on the acceleration value and the heart rate according to an embodiment.

FIG. 9 is a flowchart illustrating an operation in which the electronic device identifies the accident state, based on the distance between the first external electronic device and the second external electronic device according to an embodiment.

FIG. 10A is a flowchart illustrating an operation in which the electronic device identifies the accident state, based on an atmospheric pressure change according to an embodiment.

FIGS. 10B and 10C are diagrams illustrating an operation in which the electronic device identifies the accident state, based on an atmospheric pressure change according to an embodiment.

FIG. 11 is a diagram illustrating an operation in which the first external electronic device or the second external electronic device displays accident information of the car and an operation of asking for an emergency rescue.

FIG. 12 is a diagram illustrating an operation in which the electronic device configures a function of asking for an emergency rescue according to an embodiment.

[Mode for Carrying out the Invention]

Hereinafter, embodiments of the disclosure are described in detail to allow those skilled in the art to which the disclosure belongs to easily implement the disclosure with reference to the drawings. However, the disclosure may be implemented in various different forms and is not limited to embodiments described herein. In connection with description of the drawings, the same or similar reference numerals can be used for the same or similar elements. Further, in drawings and relevant description, description of well-known functions and configurations may be omitted for clarity and brevity.

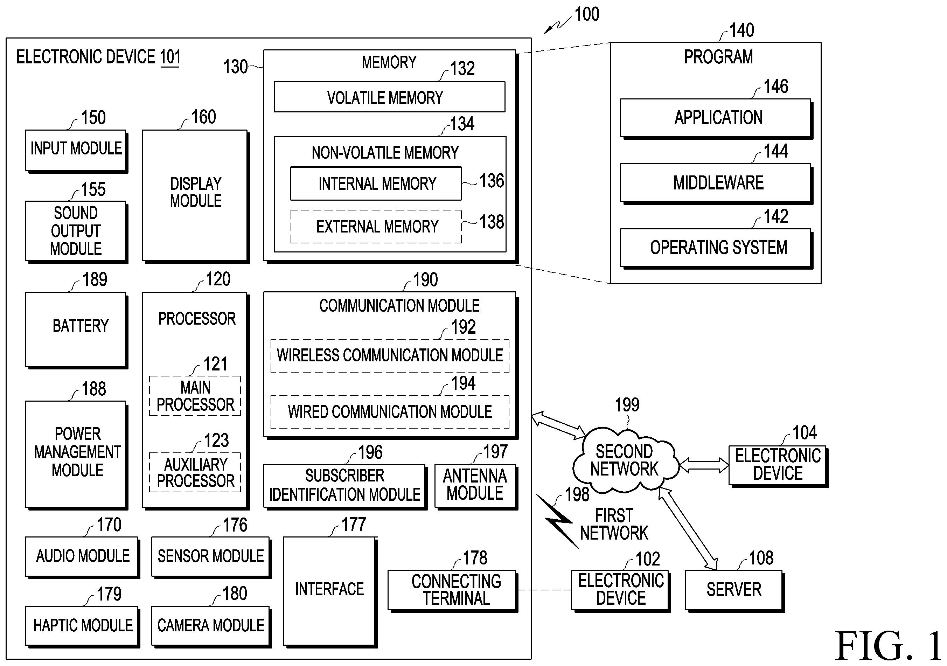

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth™, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

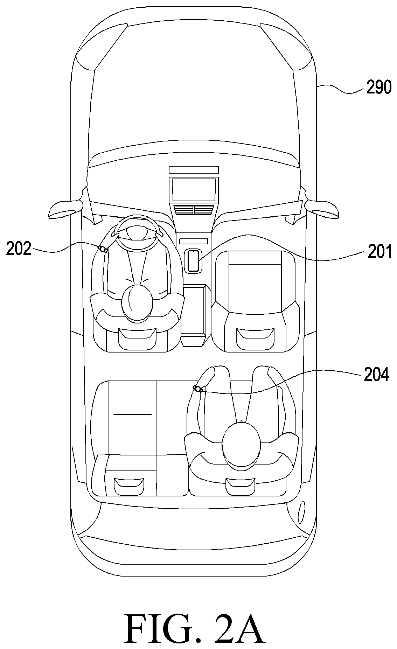

FIG. 2A is a diagram illustrating an electronic device and a first external electronic device of a user in a car, and a second external electronic device of a passenger according to an embodiment.

Referring to FIG. 2A, according to an embodiment, an electronic system may include an electronic device 201 (for example, a smartphone) (for example, the electronic device 101 of FIG. 1) and a first external electronic device 202 (for example, a smartwatch) (for example, the electronic device 102 of FIG. 1) of a user in a car 290. The electronic system may further include a second external electronic device 204 (for example, the electronic device 102 of FIG. 1) of a passenger in the car 290.

According to an embodiment, the electronic device 201 may detect collisions and/or accidents of the car 290 while the car 290 which the user is riding is driven. The electronic device 201 may detect collisions and/or accidents of the car 290 through a sensor included in the electronic device 201. However, when the electronic device 201 detects the collisions and/or accidents of the car 290 through its own sensor, the electronic device may misrecognize it. For example, when the car stops suddenly while driving or a high-decibel noise similar to an accident situation is detected, the electronic device 201 may misrecognize it as a collision situation or an accident situation of the car 290.

The electronic device 201 according to an embodiment of the disclosure may identify collisions and/or accidents of the car 290 in further consideration of information (for example, information on acceleration, atmospheric pressure, and a heart rate) sensed by the first external electronic device 202 and the second external electronic device 204 while the car 290 is driven. To this end, the electronic device 201 may configure a communication connection with each of the first external electronic device 202 and the second external electronic device 204. Further, the electronic device 201 may receive information sensed by the first external electronic device 202 and the second external electronic device 204. Accordingly, the electronic device 201 may more accurately identify the collision situation or the accident situation of the car 290.

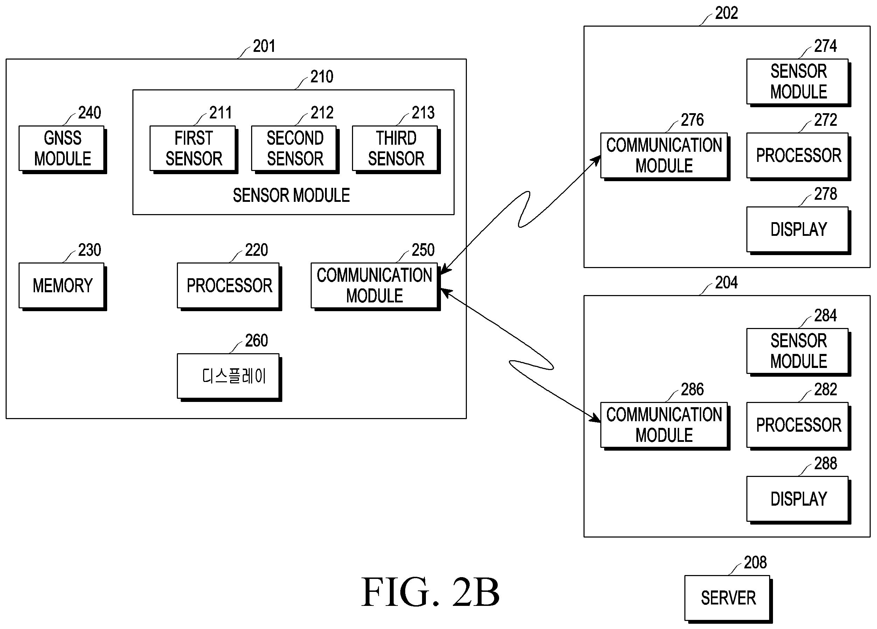

FIG. 2B is a block diagram of the electronic device, the first external electronic device, the second external electronic device, and the server according to an embodiment.

Referring to FIG. 2B, according to an embodiment, the electronic device 201 may include a sensor module 210, a processor 220, memory 230, a GNSS module 240, a communication module 250, and a display 260.

According to an embodiment, the sensor module 210 may sense information or data for identifying collisions and/or accidents of the car (for example, the car 290 of FIG. 2A). For example, the sensor module 210 may include a first sensor 211, a second sensor 212, and a third sensor 213. For example, the first sensor 211 may include an acceleration sensor for sensing acceleration. The second sensor 212 may include a sound sensor for sensing a sound. The third sensor 213 may include an atmospheric pressure sensor for sensing atmospheric pressure. Meanwhile, the sensors 211, 212, and 213 illustrated in FIG. 2B are only examples, and sensors included in the sensor module 210 may not be limited thereto.

According to an embodiment, the global navigation satellite system (GNSS) module 240 may receive a signal for identifying the location of the electronic device 201. For example, the GNSS module may receive a signal for identifying the location of the electronic device 201 by using at least one of a GPS, a GLONASS, Galileo, or Beidou.

According to an embodiment, the processor 220 may control the overall operation of the electronic device 201. For example, the processor 220 may be implemented to the same as or similar to the processor 120 of FIG. 1.

According to an embodiment, the processor 220 may configure, through the communication module 250, a first communication connection with the first external electronic device 202 worn by the user. For example, the communication module 250 may support short-range communication technology (for example, Bluetooth, Bluetooth low energy (BLE), and WiFi). Further, the communication module 250 may support ultra wide band (UWB) communication technology. The first communication connection may be configured using one of the communication technologies supported by the communication module 250. For example, the first external electronic device 202 may be implemented as a smartwatch which can be worn on a user's wrist.

According to an embodiment, the processor 220 may identify that the user is in the car 290 through at least one of the sensor module 210, the GNSS module 240, or the communication module 250. For example, the processor 220 may detect a vibration pattern of the car 290 through the first sensor 211, detect a magnetic pattern of the car through a geomagnetic sensor (not shown), detect movement at a predetermined speed or faster through the GPS module 240, or detect a pattern of an engine sound of the car 290 and/or a low-frequency sound output from the car through the second sensor 212. The processor 220 may identify or determine whether the user is in the car 290, based on the detected pattern and/or information. Further, when a connection to a set-top device (not shown) (for example, a car application installed in the set-top device) mounted to the car the electronic device 201 is made or the connection to the set-top device is made through Bluetooth communication, the electronic device 201 may determine that the user has boarded the car.

According to an embodiment, the processor 220 may identify the distance between the electronic device 201 and the second external electronic device 204. For example, the processor 201 may identify the distance between the electronic device 201 and the second external electronic device 204 and a change in the distance through the GNSS module 240. Alternatively, when the communication module 250 supports UWB communication technology, the processor 201 may identify the distance between the electronic device 201 and the second external electronic device 204, and the change in the distance through the communication module 250.

According to an embodiment, when it is identified that the distance between the electronic device 201 and the second external electronic device 204 is shorter than a predetermined distance and that the change in the distance between the electronic device 201 and the second external electronic device 204 for a predetermined time is smaller than a predetermined value, the processor 220 may identify that an owner of the second external electronic device 204 is the passenger of the car 290. For example, the predetermined distance and the predetermined value may be determined as values for identifying that the owner of the second external electronic device 204 is the passenger of the car 290. For example, the predetermined distance and the predetermined value may be automatically configured by the processor 220 or configured by the user.

According to an embodiment, when it is identified that the owner of the second external electronic device 204 is the passenger of the car, the processor 220 may attempt a second communication connection with the second external electronic device 204 of another person around the electronic device. The second communication connection may be configured using one of the communication technologies supported by the communication module 250. For example, the processor 220 may make a request for providing or sharing information sensed by the second external electronic device 204 to the second external electronic device 204. When the second external electronic device 204 agrees to provide or share the sensed information, the information sensed by the second external electronic device 204 may be received or acquired thorough the second communication connection. Alternatively, when the second external electronic device 204 does not agree to provide or share the sensed information, the processor 220 may transmit information on a message to the second external electronic device to display the message to ask for consent to a display 288 of the second external electronic device 204.

Meanwhile, the first external electronic device 202 has the same user as the electronic device 201, and thus the first external electronic device may not ask for consent to provide or share the information sensed by the first external electronic device 202. However, according to implementation, the electronic device 201 may need consent of the first external electronic device 202 to receive the information sensed by the first external electronic device 202.

According to an embodiment, the processor 220 may store, in the memory 230, information for identifying collisions and/or accidents of the car acquired through the sensor module 210. The processor 220 may store information received from the first external electronic device 202 and the second external electronic device 204 in the memory 230. Further, when the electronic device 201 configures the communication connection with each of the first external electronic device 202 and the second external electronic device 204, the processor 220 may store, in the memory 130, information on the distance between the electronic device 201 and the first external electronic device 202 and information on the distance between the electronic device 201 and the second external electronic device 204. For example, the memory 230 may be implemented to be the same as or similar to the memory 130 of FIG. 1.

According to an embodiment, after the first communication connection and the second communication connection are configured, the processor 220 may detect whether there are collisions of the car 290 through the sensor module 210 and/or the GNSS module 240.

According to an embodiment, in the state where the user is in the car 290 (for example, the state where the car 290 is being driven), when an acceleration value sensed by through the first sensor 211 is larger than or equal to a predetermined threshold, a GNSS movement speed identified through the GNSS module 240 rapidly decreases, and a sound suspected to be a collision of the car 290 is detected, the processor 220 may identify that the car 290 had a collision or an accident. Further, the processor 220 may identify whether a change in an angular velocity is larger than or equal to a predetermined threshold through the first sensor 211. For example, when a heading direction of the car 290 changes abruptly to a different direction due to a collision, the processor 220 may detect a rapid change in the angular velocity through the first sensor 211. Further, since a dominant pattern is generated in movement in a horizontal direction due to the collision of the car 290, the processor 220 may detect a change in the angular velocity by controlling or increasing a weight of movement (or impulse) in the horizontal direction of the ground of the navigation frame sensed by the first sensor 211. The processor 220 may identify whether a change in the atmospheric pressure is larger than or equal to a predetermined through the third sensor 213. For example, when windows break and the airbag deploys due to the collision of the car 290, the atmospheric pressure of the car 290 may change rapidly, and thus the processor 220 may detect a change in the atmospheric pressure. The processor 220 may detect whether the car 290 collides (or there is an accident) based on the above-described sensing values.

According to an embodiment, when detecting the collision of the car 290, the processor 220 may transmit a trigger signal to each of the first external electronic device 202 and the second external electronic device 204. For example, the trigger signal may be a signal for synchronizing the electronic device 201 and the first external electronic device 202, and the electronic device 201 and the second external electronic device 204, based on a time point at which the collision of the car is detected. At this time, the processor 220 may transmit the trigger signal and information on an acceleration value (for example, a value sensed through the first sensor 211) corresponding to the collision to the first external electronic device 202 and the second external electronic device 204.

According to an embodiment, the processor 220 may receive information sensed by the first external electronic device 202 through the first communication connection and receive information sensed by the second external electronic device 204 through the second communication connection. At this time, the sensed information may be information synchronized at a time point at which the collision of the car 290 is generated by the trigger signal. For example, the processor 220 may acquire or identify information on the acceleration, the heart rate, and the atmospheric pressure sensed by each of the first external electronic device 202 and the second external electronic device 204 through the sensed information.

According to an embodiment, the processor 220 may identify accident information indicating an accident situation by the collision of the car 290, based in the sensed information received from the first external electronic device 202 and the second external electronic device 204. At this time, the processor 220 may transmit an emergency message and/or a signal for help to a predetermined organization or a predetermined contact (for example, 119). Further, the processor 220 may transmit accident information indicating the accident situation due to the collision of the car 290 to the server 208. For example, the server 208 may be a server managed by a predetermined service provider, a server managed by an insurance company, or a server managed by a car manufacturer.

According to an embodiment, the processor 220 may display accident information through the display 260. Alternatively, the processor 220 may provide a notification informing the user of the accident through a vibration element.

According to an embodiment, the first external electronic device 202 may include the processor 272, the sensor module 274, the communication module 276, and the display 278.

According to an embodiment, the processor 272 may control the overall operation of the first external electronic device 202. The processor 272 may sense information on the acceleration, the heart rate, and the atmospheric pressure through the sensor module 274. For example, heart rate may be a heart rate of the user wearing the first external electronic device 202. The processor 272 may receive the trigger signal through the communication module 276. The processor 272 may transmit information sensed through the sensor module 274 to the electronic device 201 (or the communication module 250), based on the trigger signal being received.

According to an embodiment, the processor 272 may display the accident situation or the accident information due to the collision of the car on the display 278. At this time, the processor 272 may receive the accident information indicating the accident situation due to the collision of the car 290 from the electronic device 201 through the communication module 276. The processor 272 may transmit the emergency message and/or the signal for help to the predetermined organization or contact, based on the accident information being received. The processor 272 may transmit the accident information to the server 208.

According to an embodiment, the processor 282 may control the overall operation of the second external electronic device 204. The processor 282 may sense information on the acceleration, the heart rate, and the atmospheric pressure through the sensor module 284. For example, the heart rate may be a heart rate of the passenger wearing the second external electronic device 204. The processor 282 may receive the trigger signal through the communication module 286. The processor 282 may transmit information sensed through the sensor module 284 to the electronic device 201 (or the communication module 250), based on the trigger signal being received.

According to an embodiment, when the second external electronic device 204 does not agree to provide or share the information sensed through the sensor module 284, the processor 282 may display a message asking for consent on the display 288. For example, the message may be displayed every predetermined period and a predetermined number of times. Based on a user input for the message, the processor 282 may provide or share the information sensed through the sensor module 284. For example, when the user does not agree to provide or share the sensed information, the processor 282 may not provide the information sensed through the sensor module 284 to the electronic device 201. Alternatively, when the user agrees to provide or share the sensed information, the processor 282 may provide the information sensed through the sensor module 284 to the electronic device 201, based on the trigger signal being received.

Operations of the electronic device 201 described below may be performed by the processor 220. Further, operations of the first external electronic device 202 may be performed by the processor 272, and operations of the second external electronic device 204 may be performed by the processor 282. However, for convenience of description, it is assumed and described that the entity of the operations is the electronic device 201 (or the first external electronic device 202 or the second external electronic device 204).

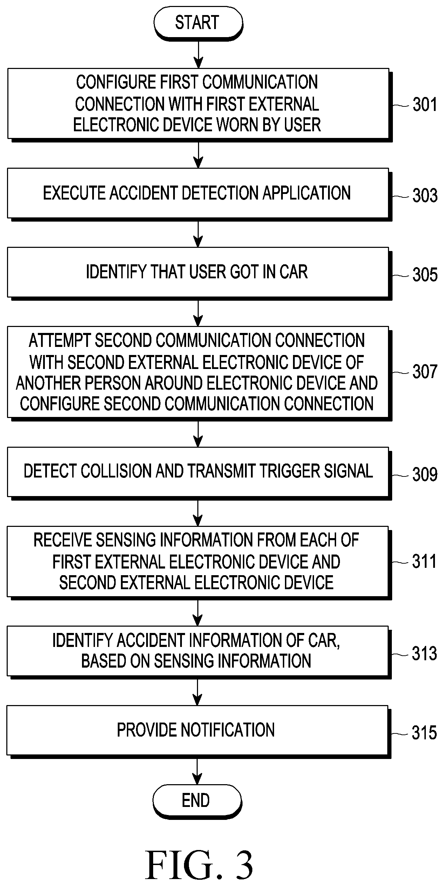

FIG. 3 is a flowchart illustrating an operation in which the electronic device identifies accident information of the car according to an embodiment.

In the following embodiments, respective operations may be sequentially performed but the sequential performance is not necessary. For example, orders of the operations may be changed, and at least two operations may be performed in parallel.

According to an embodiment, it may be understood that operations 301 to 315 are performed by the processor (for example, the processor 220 of FIG. 2B) of the electronic device (for example, the electronic device 201 of FIGS. 2A and 2B).

Referring to FIG. 3, according to an embodiment, in operation 301, the electronic device 201 may configure a first communication connection with a first external electronic device (for example, the first external electronic device 202 of FIGS. 2A and 2B) worn on the user. For example, the first communication connection may be configured using short-range communication technology (for example, Bluetooth).

According to an embodiment, in operation 303, the electronic device 201 may execute an accident detection application. For example, the accident detection application may be an application for performing a function of detecting an accident of the car 290.

According to an embodiment, in operation 305, the electronic device 201 may identify that the user is in the car. For example, the electronic device 201 may identify that the user is in the car by using at least one of a sensor module (for example, the sensor module 210 of FIG. 2B), a GNSS module (for example, the GNSS module 240 of FIG. 2B), and a communication module (for example, the communication module 250 of FIG. 2B).

According to an embodiment, in operation 307, the electronic device 201 may attempt the second communication connection with the second external electronic device (for example, the second external electronic device 204 of FIGS. 2A and 2B) of another person around the electronic device 201. The electronic device 201 may configure the second communication connection with the second external electronic device 204, based on the attempt of the second communication connection. For example, when the second external electronic device 204 agrees to provide sensing information (for example, information sensed by the second external electronic device 204) to the electronic device 201, the electronic device 201 may configure the second communication connection with the second external electronic device 204.

Meanwhile, according to implementations, the order of operations 301 to 307 is only an example and may be changed according to implementations.

According to an embodiment, in operation 309, the electronic device 201 may detect the collision of the car and transmit a trigger signal to the first external electronic device and the second external electronic device.

According to an embodiment, in operation 311, the electronic device 201 may receive information (hereinafter, referred to as sensing information) sensed by each of the first external electronic device 202 and the second external electronic device 204 from the first external electronic device 202 and the second external electronic device 204. Further, the electronic device 201 may repeatedly receive information (hereinafter, referred to as sensing information) sensed by each of the first external electronic device 202 and the second external electronic device 204. For example, the sensing information may include information on acceleration, a heart rate, atmospheric pressure, a location, gyro, and/or a sound sensed by each of the first external electronic device 202 and the second external electronic device 204. The sensing information may include information sensed by the first external electronic device 202 and the second external electronic device 204 since a predetermined time from a time at which the trigger signal is transmitted.

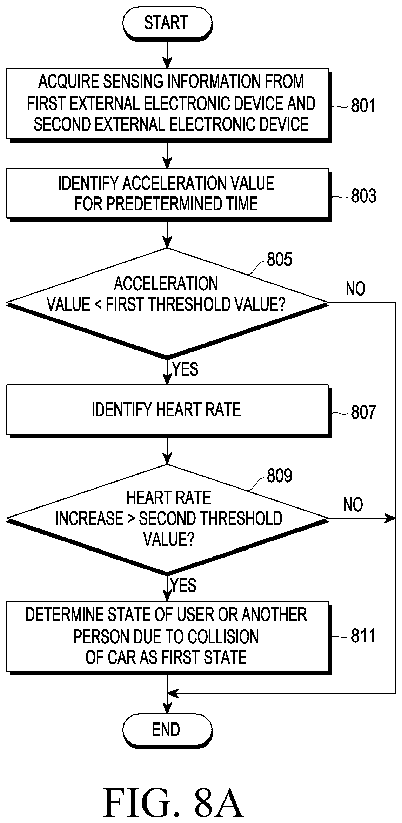

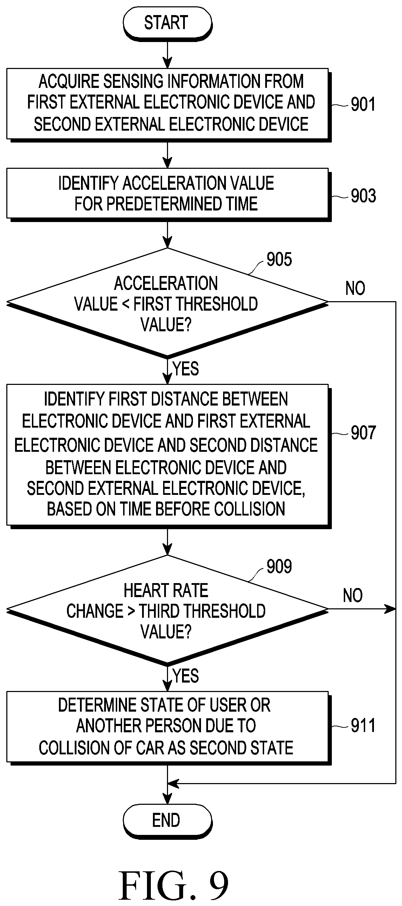

According to an embodiment, in operation 313, the electronic device 201 may identify accident information indicating a collision situation and/or an accident situation of the car 290, based on the sensing information. For example, the electronic device 201 may calculate the magnitude of a normal value of the acceleration 3-axis (for example, x axis, y axis, and z axis) and identify whether the corresponding value is larger than or equal to a predetermined magnitude (for example, a first threshold value of FIGS. 8A and 9). At this time, the electronic device 201 may perform coordinate conversion for data of the acceleration 3-axis (for example, width, length, and height) from a body frame (or a body coordinate system) to a navigation frame (or an ENU coordinate system) and identify whether impulse in an east (E) axis and an north (N) axis of the navigation frame (or the ENU coordinate system) rapidly changes to be larger than or equal to a predetermined magnitude. For example, the body frame and the navigation frame may be one of various coordinate systems of an inertial sensor. For example, the body frame is also called a body coordinate system and may be a coordinate system fixed based on the electronic device 201. For example, the acceleration 3-axis may be information output based on the body frame. For example, the navigation frame is also called an ENU coordinate system in which an E axis may mean east, an N axis may mean north, a U axis may mean up.

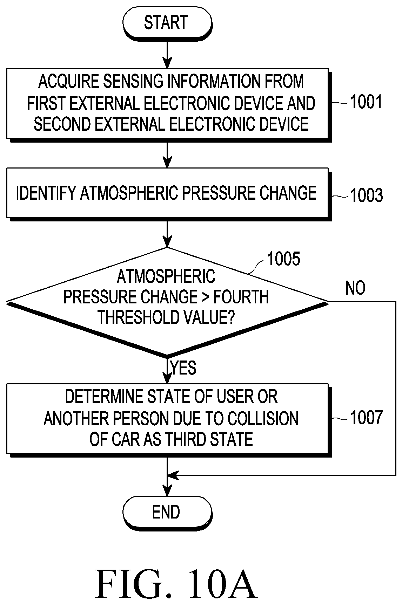

The electronic device 201 may identify whether a change in the angular velocity of a gyro sensor rapidly changes to be larger than or equal to a predetermined magnitude. The electronic device 201 may identify whether a change in the atmospheric pressure changes to be larger than or equal to a predetermined magnitude (for example, a fourth threshold value of FIG. 10A). The electronic device 201 may identify whether a movement speed and/or a movement distance rapidly decreases based on a location change. The electronic device 201 may identify whether the heart rate rapidly changes to be larger than or equal to a predetermined magnitude (for example, a second threshold value of FIG. 8A). The electronic device 201 may identify whether a pattern of acceleration and gyro corresponds to a pattern of the car collision and/or the car accident through machine learning. Further, the electronic device 201 may identify whether a sound pattern corresponds to the pattern of the car collision and/or the car accident through machine learning. The electronic device 201 may determine whether the car 290 collides and the accident situation by using at least some of the identified information. Further, the electronic device 201 may acquire accident information indicating whether the car 290 collides and the accident situation, based on the determination result.

According to an embodiment, in operation 315, the electronic device 201 may provide a notification based on the accident information. For example, the electronic device 201 may provide the notification through a sound and/or vibration. Further, the electronic device 201 may transmit an emergency message and/or a signal for help to a predetermined organization or a predetermined contact (for example, 119 or a predetermined person). Meanwhile, the first external electronic device 202 may identify the location of the user and record sounds in a surrounding situation for a predetermined time. Thereafter, the first external electronic device 202 may transmit information on the location of the user and recorded data to the predetermined organization or the predetermined contact (for example, 119 or the predetermined person).

Through the above-described method, the electronic device 201 may more accurately determine or identify the collision situation and the accident situation of the car 290.

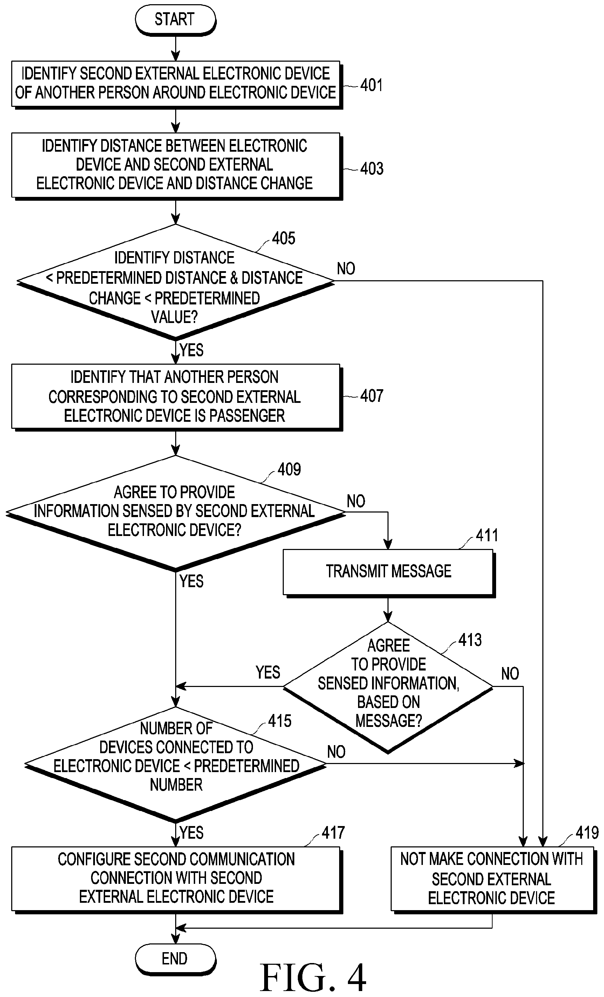

FIG. 4 is a flowchart illustrating an operation in which the electronic device receives sensing information from the surrounding second external electronic device according to an embodiment.

In the following embodiments, respective operations may be sequentially performed but the sequential performance is not necessary. For example, orders of the operations may be changed, and at least two operations may be performed in parallel.

According to an embodiment, it may be understood that operations 401 to 419 are performed by the processor (for example, the processor 220 of FIG. 2B) of the electronic device (for example, the electronic device 201 of FIGS. 2A and 2B).

Referring to FIG. 4, according to an embodiment, in operation 401, the electronic device 201 may identify the second external electronic device (for example, the second external electronic device 204 of FIGS. 2A and 2B) of another person around the electronic device 201.

According to an embodiment, in operation 403, the electronic device 201 may identify the distance between the electronic device 201 and the second external electronic device 204 and a distance change. For example, the electronic device 201 may analyze the strength of a signal between the electronic device 201 and the second external electronic device 204 and identify the distance between the electronic device 201 and the second external electronic device 204. Alternatively, when the electronic device 201 and the second external electronic device 204 support UWB technology, the electronic device 201 may identify the distance between the electronic device 201 and the second external electronic device 204 by using UWB technology. Alternatively, the electronic device 201 may identify the distance between the electronic device 201 and the second external electronic device 204 by using a GNSS signal. The electronic device 201 may identify a change in the distance between the electronic device 201 and the second external electronic device 204 for a predetermined time. For example, the predetermined time may be a time for identifying whether an owner of the second external electronic device 204 is the passenger of the car (for example, the car 290 of FIG. 2A). For example, the predetermined time may be automatically configured by the electronic device 201 or may be configured by the user.

According to an embodiment, in operation 405, the electronic device 201 may identify whether the distance between the electronic device 201 and the second external electronic device 204 is shorter than a predetermined distance and the change in the distance between the electronic device 201 and the second external electronic device 204 for the predetermined time is smaller than a predetermined value.

According to an embodiment, when it is identified that the distance between the electronic device 201 and the second external electronic device 204 is shorter than the predetermined distance and the change in the distance between the electronic device 201 and the second external electronic device 204 for the predetermined time is smaller than the predetermined value (Yes of operation 405), the electronic device 201 may identify that the other person corresponding to the second external electronic device 204 (for example, the owner of the second external electronic device 204) is the passenger of the car 290 in operation 407.

According to an embodiment, when the distance between the electronic device 201 and the second external electronic device 204 is not shorter than the predetermined distance or the change in the distance between the electronic device 201 and the second external electronic device 204 for the predetermined time is not smaller than the predetermined value (No of operation 405), the electronic device 201 may not configure a communication connection with the second external electronic device 204 in operation 419. For example, the electronic device 201 may determine that the owner of the second external electronic device 204 is not the passenger of the car 290. At this time, information sensed by the second external electronic device 204 may not be provided to the electronic device 201.

According to an embodiment, in operation 409, the electronic device 201 may identify whether the second external electronic device 204 agrees to provide the sensed information.

According to an embodiment, when the second external electronic device 204 does not agree to provide the sensed information (No of operation 409), the electronic device 201 may transmit a message for asking for consent to the second external electronic device 204 in operation 411.

According to an embodiment, in operation 413, the electronic device 201 may identify whether the second external electronic device 204 agrees to provide the sensed information, based on the message being transmitted.

According to an embodiment, when the second external electronic device 204 does not agree to provide the sensed information (No of operation 413), the electronic device 201 may not configure the communication connection with the second external electronic device 204 in operation 419. At this time, the information sensed by the second external electronic device 204 may not be provided to the electronic device 201.

According to an embodiment, when the second external electronic device 204 agrees to provide the sensed information (Yes of operation 413), the electronic device 201 may identify whether the number of devices currently connected to the electronic device 201 is smaller than a predetermined number in operation 415. For example, when the predetermined number is 3, the electronic device 201 may identify whether the number of external devices currently connected to the electronic device 201 is smaller than 3.

According to an embodiment, when it is identified that the number of devices currently connected to the electronic device 201 is smaller than the predetermined number (Yes of operation 415), the electronic device 201 may configure a second communication connection with the second external electronic device in operation 417. Thereafter, when the collision of the car 290 is detected, the electronic device 201 may receive sensing information from the second external electronic device 204.

According to an embodiment, when it is identified that the number of devices connected to the electronic device 201 is not smaller than the predetermined number (No of operation 415), the electronic device 201 may not configure the communication connection with the second external electronic device 204. At this time, the information sensed by the second external electronic device 204 may not be provided to the electronic device 201. Thereafter, when the number of devices connected to the electronic device 201 becomes smaller than the predetermined number, the electronic device 201 may re-attempt the connection with the second external electronic device 204.

Meanwhile, when the second external electronic device 204 is a predetermined device (for example, a predetermined user (for example, a family member or a relative), the electronic device 201 may omit at least some of operations 401 to 417 described above.

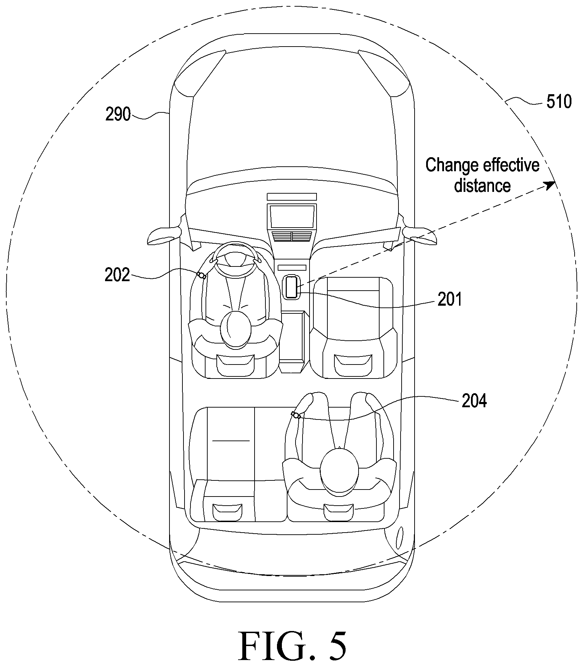

FIG. 5 is a diagram illustrating an operation in which the electronic device receives sensing information from the surrounding second external electronic device according to an embodiment.

Referring to FIG. 5, the electronic device 201 may attempt a second communication connection with the second external electronic device 204, based on the distance between the electronic device 201 and the second external electronic device 204 being shorter than a predetermined distance. For example, the electronic device 201 may attempt the connection with at least one external electronic device (for example, the second external electronic device 204) located in an area 510 having a radius of a predetermined distance (or an effective distance) from the location of the electronic device 201. For example, the predetermined distance may be determined based on a distance at which both the user and the passenger can is in the car 290.

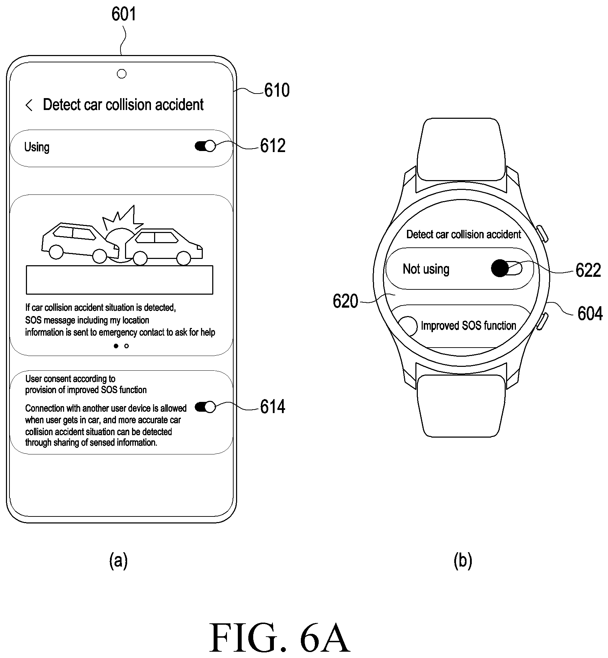

FIG. 6A is a diagram illustrating an operation in which the electronic device and the second external electronic device perform an accident detection function of the car according to an embodiment.

Referring to part (a) of FIG. 6A, according to an embodiment, an electronic device 601 (for example, the electronic device 201 of FIG. 2B) may display a screen 610 for performing a function of detecting a collision and/or an accident situation of the car 290. For example, the electronic device 201 may perform the function of detecting the collision and/or the accident situation of the car 290, based on a user input for a first object 612. At this time, the electronic device 201 may share information sensed by a sensor module (for example, the sensor module 210 of FIG. 2B) with another electronic device (for example, the second external electronic device 204 or 604) connected to communicate with the electronic device 601, based on a user input for a second object 614. For example, when the second object 614 changes to an on state (for example, moves to the right side), the electronic device 601 may share information sensed by the sensor module 210 with another electronic device (for example, the second external electronic device 604) connected to communicate with the electronic device 601.

Referring to part (b) of FIG. 6A, according to an embodiment, the second external electronic device 604 (for example, the second external electronic device 204 of FIG. 2B) may display a screen 620 for performing an accident detection function of the car. For example, based on a user input for a third object 622, information sensed by a sensor module (for example, the sensor module 284 of FIG. 2B) may be shared with another electronic device (for example, the electronic device 201 or 601) connected to communicate with the second external electronic device 604. For example, when the third object 622 changes to an on state (for example, moves to the right side), the second external electronic device 604 may share information sensed by the sensor module 282 with another electronic device (for example, the electronic device 601) connected to communicate with the electronic device 604.

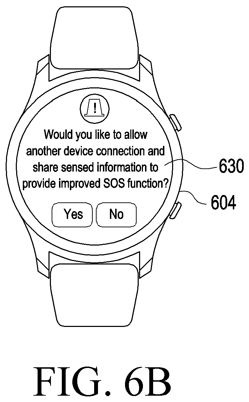

FIG. 6B is a diagram illustrating an operation in which the second external electronic device displays a message for sharing sensing information according to an embodiment.

Referring to FIG. 6B, according to an embodiment, the electronic device 201 or 601 may identify whether the second external electronic device 604 agrees to provide sensed information.

According to an embodiment, when the second external electronic device 604 does not agree to provide sensed information (for example, maintain the third object 622 of FIG. 6A in the off state), the electronic device 201 or 601 may transmit information on a message for asking for consent to the second external electronic device 604. The second external electronic device 604 may display a message 630 on a display (for example, the display 288 of FIG. 2B), based on the information on the message. For example, the second external electronic device 604 may display the message 630 through a popup window.

According to an embodiment, the second external electronic device 604 may provide or share information sensed by the second external electronic device 604 to or with the electronic device 201 or 601, based on a user input for the message 630. For example, when a user input for "consent" included in the message 630 is identified, the second external electronic device 604 may provide or share the sensed information to or with the electronic device 201 or 601. Alternatively, when a user input for "cancel" included in the message 630 is identified, the second external electronic device 604 may not provide or share the sensed information to or with the electronic device 201 or 601.

According to an embodiment, when the electronic device 201 or 601 attempts the connection, the second external electronic device 604 may display the message 630. According to implementation, when the second external electronic device 604 does not agree to provide the sensed information, the second external electronic device 604 may display the message 630 at every designated time.

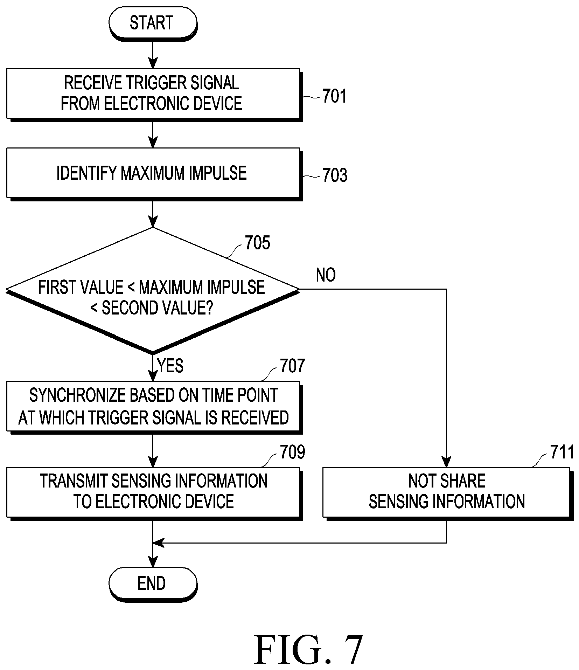

FIG. 7 is a flowchart illustrating an operation in which the first external electronic device or the second external electronic device is synchronized with the electronic device, based on a trigger signal according to an embodiment.

In the following embodiments, respective operations may be sequentially performed but the sequential performance is not necessary. For example, orders of the operations may be changed, and at least two operations may be performed in parallel.

According to an embodiment, it may be understood that operations 701 to 711 are performed by a processor (for example, the processor 272 or the processor 282 of FIGS. 2A and 2B) of a first external electronic device (for example, the first external electronic device 202 of FIGS. 2A and 2B) or a second external electronic device (for example, the second external electronic device 204 of FIGS. 2A and 2B).

Referring to FIG. 7, according to an embodiment, in operation 701, the first external electronic device 202 or the second external electronic device 204 may receive a trigger signal from the electronic device 201. For example, the first external electronic device 202 and/or the second external electronic device 204 may acquire the trigger signal and information on impulse by impact of a car (for example, the car 290 of FIG. 2A) sensed by the electronic device 201. According to implementation, the information on the impulse may be included in the trigger signal.

According to an embodiment, in operation 703, the first external electronic device 202 or the second external electronic device 204 may identify maximum impulse sensed based on a time point at which the trigger signal is received.

According to an embodiment, in operation 705, the first external electronic device 202 or the second external electronic device 204 may identify whether the identified maximum impulse is a value between a first value and a second value. For example, the first value and the second value may be determined based on the impulse received from the electronic device 201. For example, the first value may be a value obtained by subtracting a predetermined offset from the impulse received from the electronic device 201, and the second value may be a value obtained by adding the predetermined offset to the impulse received from the electronic device 201. Through the operation, the first external electronic device 202 or the second external electronic device 204 may identify whether impact similar to the electronic device 201 is sensed.

According to an embodiment, when the identified maximum impulse is the value between the first value and the second value (Yes of operation 705), the first external electronic device 202 or the second external electronic device 204 may be synchronized with the electronic device 201, based on the time point at which the trigger signal is received in operation 707. The first external electronic device 202 or the second external electronic device 204 may transmit or share sensing information to or with the electronic device 201. At this time, the transmitted or provided sensing information may be information synchronized at the time point at which the trigger signal is received.

According to an embodiment, when the identified maximum impulse is not the value between the first value and the second value (No of operation 705), the first external electronic device 202 or the second external electronic device 204 may not share or provide the sensing information with or to the electronic device 201 in operation 711.

According to the above-described method, the electronic device 201 may acquire, from the first external electronic device 202 and/or the second external electronic device 204, sensing information synchronized at the time point at which the trigger signal is transmitted.

In FIGS. 8A to 10B below, the method by which the electronic device 201 identifies the collision state and/or the accident state of the car 290 described in operation 313 of FIG. 3 is described in detail.

FIG. 8A is a flowchart illustrating an operation in which the electronic device identifies the accident state, based on an acceleration value and a heart rate according to an embodiment.

In the following embodiments, respective operations may be sequentially performed but the sequential performance is not necessary. For example, orders of the operations may be changed, and at least two operations may be performed in parallel.

According to an embodiment, it may be understood that operations 801 to 811 are performed by a processor (for example, the processor 220 of FIG. 2B) of an electronic device (for example, the processor 201 of FIGS. 2A and 2B).

Referring to FIG. 8A, according to an embodiment, in operation 801, the electronic device 201 may acquire sensing information from a first external electronic device (for example, the first external electronic device 202 of FIGS. 2A and 2B) and a second external electronic device (for example, the second external electronic device 204 of FIGS. 2A and 2B). For example, the sensing information may include information on acceleration, a heart rate, and/or atmospheric pressure sensed by each of the first external electronic device 202 and the second external electronic device 204. For example, the information on the heart rate may include information on a heart rate of a user generated by the first external electronic device 202 and information on a heart rate of a passenger sensed by the second external electronic device 204.

According to an embodiment, in operation 803, the electronic device 201 may identify the acceleration value sensed by the first external electronic device 202 and the second external electronic device 204 for a predetermined time, based on the sensing information. For example, the predetermined time may be a predetermined time interval after the trigger signal is transmitted. For example, the acceleration value may be the average of magnitude of the acceleration 3-axis (x axis, y axis, and z axis) sensed for a predetermined time.

According to an embodiment, in operation 805, the electronic device 201 may identify whether the acceleration value is smaller than a first threshold value. For example, the first threshold value may be configured as a value for determining the state in which the user is not moving or is barely moving (for example, the unconscious state). When the acceleration value is smaller than the first threshold value, the electronic device 201 may determine the state in which the user is not moving or is barely moving (for example, the unconscious state).

According to an embodiment, when it is identified that the acceleration value is smaller than the first threshold value (Yes of operation 805), the electronic device 201 may identify the heart rate sensed by the first external electronic device 202 and/or the second external electronic device 204 in operation 807.

According to an embodiment, in operation 809, the electronic device 201 may identify whether an increase in the heart rate is larger than a second threshold value. For example, the electronic device 201 may identify whether there is a rapid heart rate increase for a predetermined time from the time point at which the trigger signal is transmitted. For example, when there is strong chest compression due to a collision of the car 290 in the state where the user is wearing a seat belt provided in the car (for example, the car 290 of FIG. 2A), the heart rate may temporarily increase. The second threshold value may be configured as a value for determining the state in which there is strong chest compression due to the collision of the car 290 in the state where the user is wearing the seat belt provided in the car 290.

According to an embodiment, when it is identified that the heart rate increase is larger than the second threshold value (Yes of operation 809), the electronic device 201 may determine the state of the user or another person due to the collision of the car 290 as a first state in operation 811. For example, the first state may be a state in which a collision accident of the car 290 is generated while the user or the other person is wearing the seat belt. For example, when the acceleration value and the heart rate value sensed by the first external electronic device 202 meet the conditions of operations 805 and 809, the electronic device 201 may determine that the user is in the first state. Further, when the acceleration value and the heart rate value sensed by the second external electronic device 204 meet the conditions of operations 805 and 809, the electronic device 201 may determine that the other person (or the passenger) is in the first state. At this time, the electronic device 201 may determine a probability value indicating possibility of the first state.

According to an embodiment, when it is identified that the acceleration value is not smaller than the first threshold value (No of operation 805) or the heart rate increase is not larger than the second threshold value (No of operation 809), the electronic device 201 may determine that the state of the user or the other person due to the collision of the car is not the first state. According to implementation, when it is identified that the acceleration value is smaller than the first threshold value (Yes of operation 805) and it is identified that the heart rate increase is not larger than the second threshold value (No of operation 809), the electronic device 201 may determine that the state of the user or the other person due to the collision of the car is in the first state. At this time, the electronic device 201 may determine a probability value indicating possibility of the first state.

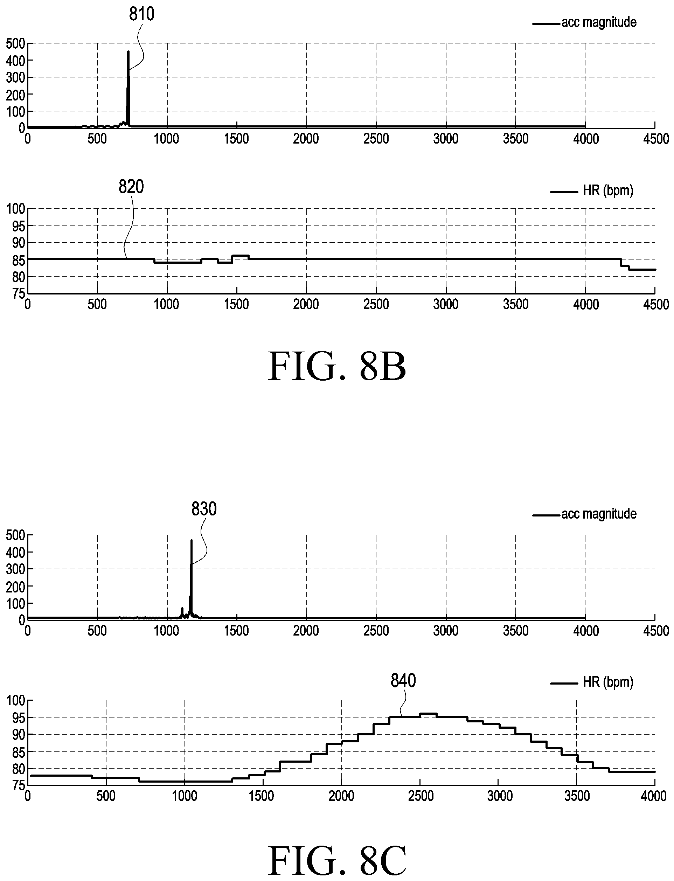

FIGS. 8B and 8C are diagrams illustrating an operation in which the electronic device identifies the accident state, based on the acceleration value and the heart rate according to an embodiment.

Referring to FIG. 8B, according to an embodiment, a first graph 810 may show an acceleration value (or acceleration impulse) sensed by the first external electronic device 202 or the second external electronic device 204. For example, the x axis of the first graph 810 may indicate the time or the number of accumulated samples determined according to the time. The y axis of the first graph 810 may indicate an acceleration value (for example, $m/s^2$). A second graph 820 may indicate a heart rate sensed by the first external electronic device 202 or the second external electronic device 204. For example, the x axis of the second graph 820 may indicate the time or the number of accumulated samples determined according to the time. The y axis of the second graph 820 may indicate a heart rate value (for example, bpm).

According to an embodiment, the first graph 810 may indicate that the acceleration value temporarily increases when impact (for example, impulse of about 40 G) is generated by the first external electronic device 202 or the second external electronic device 204. The second graph 820 may indicate that there is no large difference between heart rates before and after the impact is generated.

Referring to FIG. 8C, a third graph 830 may indicate an acceleration value (or acceleration impulse) sensed by the first external electronic device 202 or the second external electronic device 204. For example, the x axis of the third graph 830 may indicate the time or the number of accumulated samples determined according to the time. The y axis of the third graph 830 may indicate an acceleration value (for example, $m/s^2$). A fourth graph 840 may indicate a heart rate sensed by the first external electronic device 202 or the second external electronic device 204. For example, the x axis of the fourth graph 840 may indicate the time or the number of accumulated samples determined according to the time. The y axis of the fourth graph 840 may indicate a heart rate value (for example, bpm).

According to an embodiment, the third graph 830 may indicate that the acceleration value temporarily increases when strong impact (for example, impulse of about 45 G) occurs on the first external electronic device 202 or the second external electronic device 204. The fourth graph 840 may indicate a pattern in which the heart rate temporarily increases significantly and then decreases after impact is generated.

According to an embodiment, when the acceleration value and the heart rate are identified as illustrated in FIG. 8C, the electronic device 201 may determine the state of the user or the other person due to the collision of the car 290 as the first state.

According to an embodiment, when the acceleration value and the heart rate are identified as illustrated in FIG. 8B, the electronic device 201 may determine that the state of the user or the other person due to the collision of the car 290 is not the first state. Alternatively, when the acceleration value and the heart rate are identified as illustrated in FIG. 8B, the electronic device 201 may determine a probability value indicating that the state of the user or the other person due to the collision of the car 290 may be the first state.

FIG. 9 is a flowchart illustrating an operation in which the electronic device identifies an accident state, based on the distance between the first external electronic device and the second external electronic device according to an embodiment.

In the following embodiments, respective operations may be sequentially performed but the sequential performance is not necessary. For example, orders of the operations may be changed, and at least two operations may be performed in parallel.

According to an embodiment, it may be understood that operations 901 to 911 are performed by a processor (for example, the processor 220) of an electronic device (for example, the processor 201 of FIGS. 2A and 2B).

Referring to FIG. 9, according to an embodiment, in operation 901, the electronic device 201 may acquire sensing information from a first external electronic device (for example, the first external electronic device 202 of FIGS. 2A and 2B) and a second external electronic device (for example, the second external electronic device 204 of FIGS. 2A and 2B). For example, the sensing information may include information on acceleration and a location (or the distance from the electronic device 201) sensed by each of the first external electronic device 202 and the second external electronic device 204. For example, the information on the distance may include information on a first distance between the electronic device 201 and the first external electronic device 202 and a second distance between the electronic device 201 and the second external electronic device 204.

According to an embodiment, in operation 903, the electronic device 201 may identify the acceleration value sensed by the first external electronic device 202 and the second external electronic device 204 for a predetermined time, based on the sensing information. For example, the predetermined time may be a predetermined time interval after the trigger signal is transmitted. For example, the acceleration value may be the average of magnitude of the acceleration 3-axis (x axis, y axis, and z axis) sensed for a predetermined time.

According to an embodiment, in operation 905, the electronic device 201 may identify whether the acceleration value is smaller than a first threshold value. For example, the first threshold value may be configured as a value for determining the state in which the user is not moving or is barely moving (for example, the unconscious state). When the acceleration value is smaller than the first threshold value, the electronic device 201 may determine the state in which the user is not moving or is barely moving (for example, the unconscious state).

According to an embodiment, when it is identified that the acceleration value is smaller than the first threshold value (Yes of operation 905), the electronic device 201 may identify the first distance between the electronic device 201 and the first external electronic device 202 and the second distance between the electronic device 201 and the second external electronic device 204 in operation 907.

According to an embodiment, in operation 907, the electronic device 201 may identify the first distance between the electronic device 201 and the first external electronic device 202 and the second distance between the electronic device 201 and the second external electronic device 204 before the collision (or before the collision is detected). For example, the electronic device 201 may compare the distance between the electronic device 201 and the first external electronic device 202 before the collision is detected with the first distance after the collision is detected. Further, the electronic device 201 may compare the distance between the electronic device 201 and the second external electronic device 204 before the collision is detected and the second distance after the collision is detected. For example, the electronic device 201 may store in advance the distance between the electronic device 211 and the first external electronic device 202 and the distance between the electronic device 201 and the second external electronic device 204 before the collision is detected.

According to an embodiment, in operation 909, the electronic device 201 may identify whether a distance change after the collision (for example, a distance change of the first external electronic device 202 or a distance change of the second external electronic device 204) is larger than a third threshold value. For example, the third threshold value may be configured as a value for determining that the location of the user or another person is significantly changed due to the collision of the car 290.

According to an embodiment, when it is identified that the distance change after the collision (for example, the distance change of the first external electronic device 202 or the distance change of the second external electronic device 204) is larger than the third threshold value (Yes of operation 909) after the collision, the electronic device 201 may determine the state of the user or the other person (or the passenger) due to the collision of the car 290 is a second state in operation 911. At this time, the electronic device 201 may determine a probability value indicating possibility of the second state. For example, the second state may be a state in which the location of the user or the other person (or the passenger) is significantly changed due to the collision or accident of the car 290 while the user or the other person (or the passenger) is not wearing a seat belt. Alternatively, the second state may be a state in which the location of the electronic device 201 is significantly changed due to the collision or accident of the car 290.

According to an embodiment, when it is identified that the acceleration value is not smaller than the first threshold value (No of operation 905) or it is identified that the distance change is not larger than the third threshold value (No of operation 909), the electronic device 201 may determine that the state of the user or the other person due to the collision of the car is not the second state. According to implementation, when it is identified that the acceleration value is smaller than the first threshold value (Yes of operation 905) and it is identified that the distance change is not larger than the third threshold value (No of operation 909), the electronic device 201 may determine that the state of the user or the other person due to the collision of the car is likely to be the second state. At this time, the electronic device 201 may determine a probability value indicating possibility of the second state.

FIG. 10A is a flowchart illustrating an operation in which the electronic device identifies an accident state, based on an atmospheric pressure change according to an embodiment.

In the following embodiments, respective operations may be sequentially performed but the sequential performance is not necessary. For example, orders of the operations may be changed, and at least two operations may be performed in parallel.

According to an embodiment, it may be understood that operations 1001 to 1007 are performed by a processor (for example, the processor 220) of an electronic device (for example, the processor 201 of FIGS. 2A and 2B).

Referring to FIG. 10A, according to an embodiment, in operation 1001, the electronic device 201 may acquire sensing information from a first external electronic device (for example, the first external electronic device 202 of FIGS. 2A and 2B) and a second external electronic device (for example, the second external electronic device 204 of FIGS. 2A and 2B). For example, the sensing information may include information on acceleration, a heart rate, and/or atmospheric pressure sensed by each of the first external electronic device 202 and the second external electronic device 204. For example, the information on the atmospheric pressure may include atmospheric pressure information sensed by the first external electronic device 202 and atmospheric pressure information sensed by the second external electronic device 204.

According to an embodiment, in operation 1003, the electronic device 201 may identify an atmospheric pressure change for a predetermined time, based on sensing information. For example, the electronic device 201 may identify whether atmospheric pressure rapidly changes after a collision is detected.

According to an embodiment, in operation 1005, the electronic device 201 may identify whether the atmospheric pressure change is larger than a fourth threshold value. For example, the fourth threshold value may be configured as a value for determining that windows of the car 290 break or the airbag deploys. For example, when the windows of the car 290 break, atmospheric pressure inside the car 290 may decrease momentarily. Alternatively, when the airbag of the car 290 deploys, atmospheric pressure inside the car 290 may increase momentarily.

According to an embodiment, when it is identified that the atmospheric pressure change (for example, an atmospheric pressure change sensed by the first external electronic device 202 or an atmospheric pressure change sensed by the second external electronic device 204) is larger than the fourth threshold value (Yes of operation 1005), the electronic device 201 may determine the state of the user or the other person (or the passenger) due to the collision of the car 290 as a third state in operation 1007. At this time, the electronic device 201 may determine a probability value indicating possibility of the third state. For example, the third state may be a state in which the user or the other person (or the passenger) receives strong impact due to the collision or accident of the car 290 where the user or the other person (or the passenger) is not wearing a seat belt. Alternatively, the third state may be a state in which the first external electronic device 202 or the second external electronic device 204 receives strong impact due to the collision or accident of the car 290.

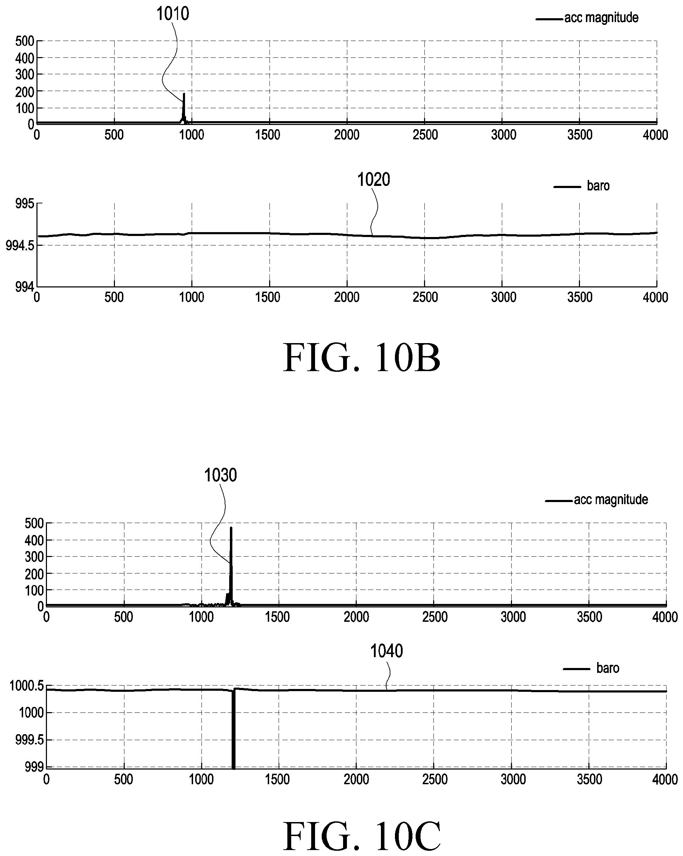

FIGS. 10B and 10C are diagrams illustrating an operation in which the electronic device identifies the accident state, based on an atmospheric pressure change according to an embodiment.

Referring to FIG. 10B, according to an embodiment, the first graph 1010 may show an acceleration value (or acceleration impulse) sensed by the first external electronic device 202 or the second external electronic device 204. For example, the x axis of the first graph 1010 may indicate the time or the number of accumulated samples determined according to the time. The y axis of the first graph 1010 may indicate an acceleration value (for example, $m/s^2$). The second graph 1020 may indicate atmospheric pressure sensed by the first external electronic device 202 or the second external electronic device 204. For example, the x axis of the second graph 1020 may indicate the time or the number of accumulated samples determined according to the time. The y axis of the second graph 1020 may indicate an atmospheric pressure value (for example, hPa).

According to an embodiment, the first graph 1010 may indicate that the acceleration value temporarily increases when somewhat weak impact (for example, impulse of about 20 G) is generated in the first external electronic device 202 or the second external electronic device 204. The second graph 1020 may indicate that there is no large difference between atmospheric pressure between and after the collision occurs.

Referring to FIG. 10C, a third graph 1030 may indicate an acceleration value (or acceleration impulse) sensed by the first external electronic device 202 or the second external electronic device 204. For example, the x axis of the third graph 1030 may indicate the time or the number of accumulated samples determined according to the time. The y axis of the third graph 1030 may indicate an acceleration value (for example, $m/s^2$). The fourth graph 1040 may indicate atmospheric pressure sensed by the first external electronic device 202 or the second external electronic device 204. For example, the x axis of the fourth graph 1040 may indicate the time or the number of accumulated samples determined according to the time. The y axis of the fourth graph 1040 may indicate an atmospheric pressure value (for example, hPa).

According to an embodiment, the third graph 1030 may indicate that the acceleration value temporarily increases significantly when strong impact (for example, impulse of about 50 G) is generated in the first external electronic device 202 or the second external electronic device 204. The fourth graph 1040 may indicate a pattern in which atmospheric pressure temporarily decreases and then returns to the original atmospheric pressure after the collision occurs.

According to an embodiment, when the acceleration value and the atmospheric pressure are identified as illustrated in FIG. 10C, the electronic device 201 may determine the state of the user or the other person due to the collision of the car 290 as the third state.

According to an embodiment, when the acceleration value and the atmospheric pressure are identified as illustrated in FIG. 10B, the electronic device 201 may determine that the state of the user of the other person due to the collision of the car 290 is not the third state. Alternatively, when the acceleration value and the atmospheric pressure are identified as illustrated in FIG. 10B, the electronic device 201 may determine a probability value indicating that the state of the user or the other person due to the collision of the car 290 may be the third state.

According to an embodiment, as described above, the electronic device 201 may identify the accident state or the accident situation due to the collision of the car 290, based on the determination result made in FIGS. 8A to 10C. For example, the electronic device 201 may determine a probability value of the first state, a probability value of the second state, and a probability value of the third state, based on sensing information received from the first external electronic device 220 and the second external electronic device 204. At this time, the electronic device 201 may determine weights of the first state, the second state, and the third state in consideration of a type of the car 290, states (for example, a sitting position) of the user and the other person (or the passenger), a driving time of the car 290, and the age of the user and the other person. For example, the electronic device may identify the accident state or the accident situation of the car 290, based on [equation 1]. For example, P(A) is a probability value of the first state, and w1 may be a weight of the first state. P(B) is a probability of the second state, and w2 may be a weight of the second state. P(C) is a probability value of the third state, and w3 may be a weight of the third state. Y may be a value indicating the accident state or the accident situation due to the collision of the car 290.

[Equation 1]

$$Y = P(A) \times w1 + P(B) \times w2 + P(C) \times w3$$

[Equation 1] above is merely an example for helping understanding and is not limited thereto, and may be modified, applied, or expanded in various ways.

For example, according to [Equation 1], when the probability value P(A) is 0.6, P(B) is 0.4, and P(C) is 0.7, the weight w1 is 0.5, w2 is 0.3, and w3 is 0.2, Y may be 0.56. When Y is larger than or equal to 0.5, the electronic device 201 may determine the accident state of the user or the other person is a fall. Alternatively, when Y is smaller than 0.5, the electronic device 201 may determine that the accident state of the user or the other person is not the fall (or non-fall.

Meanwhile, the method of determining the accident state by [Equation 1] above is only an example, and the technical idea of the disclosure is not limited thereto. For example, the electronic device 201 may determine various types of accident state, based on the above-described method.

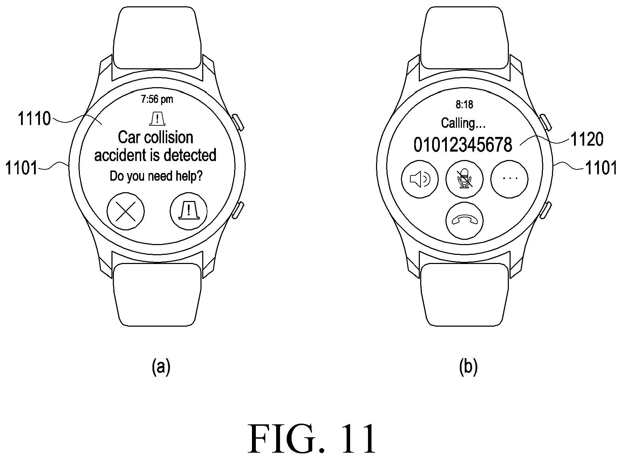

FIG. 11 is a diagram illustrating an operation in which the first external electronic device or the second external electronic device displays accident information of the car and an operation of asking for an emergency rescue according to an embodiment.

Referring to FIG. 11, according to an embodiment, when a collision and/or an accident of a car (for example, the car 290 of FIG. 2A) is identified by an electronic device (for example, the electronic device 201 of FIG. 2B), a first external electronic device 1101 (or a second external electronic device) (for example, the first external electronic device 202 or the second external electronic device 204 of FIG. 2B) may receive a signal indicating the generation of the accident from the electronic device 201. Meanwhile, when the collision and/or accident of the car 290 is identified by a plurality of electronic devices, an electronic device that first identified the generation of the accident among the plurality of electronic devices may transmit a signal indicating the generation of the accident to the first external electronic device 1101 (or the second external electronic device).

Referring to part (a) of FIG. 11, according to an embodiment, the first external electronic device 1101 (or the second external electronic device) may display a screen 1110 indicating the generation of the accident when receiving the signal indicating the generation of the accident. At this time, the first external electronic device 1101 (or the second external electronic device) may also output an alarm (for example, a SOS alarm) through a sound or vibration. According to implementation, the first external electronic device 1101 (or the second external electronic device) may perform a function of recording a surrounding situation for a predetermined time. Further, the first external electronic device 1101 (or the second external electronic device) may transmit recorded data to a predetermined contact or organization.

Referring to part (b) of FIG. 11, according to an embodiment, the first external electronic device 1101 (or the second external electronic device) may make a phone call to the predetermined contact when receiving the signal indicating the generation of the accident. At this time, the electronic device 1101 may display a screen 1120 for making the phone call. For example, the predetermined contact may be a contact designated by the user or a contact of the predetermined organization (for example, the fire station, the police station, an insurance company, or a car manufacturer).

Meanwhile, although FIG. 11 illustrates only an operation in which the first external electronic device 1101 (or the second external electronic device) outputs the notification screen or the alarm or makes the phone call to the predetermined contact, the electronic device 201 may perform an operation that is the same as or similar to the operation.

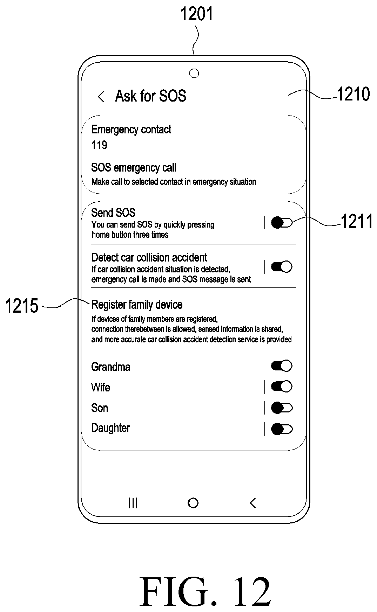

FIG. 12 is a diagram illustrating an operation in which the electronic device configures a function of asking for an emergency rescue according to an embodiment.

Referring to FIG. 12, an electronic device 1201 (for example, the electronic device 201 of FIG. 2B) may ask for an emergency rescue when the generation of an accident is identified. For example, the electronic device 1201 may configure in advance a target which the electronic device asks for the emergency rescue.

According to an embodiment, the electronic device 1201 may display a configuration screen 1210 to ask for the emergency rescue. For example, the configuration screen may include a first object 1211 for performing an emergency rescue function and a configuration window 1215 for registering in advance an external electronic device of another person.

According to a user input for the first object 1211 included in the configuration screen 1210, the electronic device 1201 may perform a function of asking for the emergency rescue. For example, when the first object 1211 is changed to the on state, the electronic device 1201 may ask the emergency contact (for example, 119) for the emergency rescue if the generation of the accident is identified.

According to an embodiment, through the operation of registering in advance the external electronic device of the other person (for example, the second external electronic device 204 of FIG. 2B), the electronic device 1201 may receive information sensed by the corresponding external electronic device when a collision is detected. At this time, through the operation of registering the external electronic device of the other person in the electronic device 1201, the electronic device 1201 may receive in advance the consent to provision of the information sensed by the corresponding external electronic device. For example, through the operation of registering in advance electronic devices of family members, the electronic device 1201 may receive in advance the consent to provision of the information sensed by the corresponding electronic devices. At this time, the configuration window 1215 may display information on external electronic devices registered in advance in the electronic device 1201. According to implementation, the electronic device 1201 may share sensed information with an external electronic device selected by the user among a plurality of external electronic devices registered in advance in the configuration window 1215.

The electronic device 201 according to an embodiment may include the sensor module 210, the GNSS module 240, the communication module 250, and the processor 220. The processor according to an embodiment may be configured to configure a first communication connection with a first external electronic device 202 worn by a user through the communication module. The processor according to an embodiment may be configured to, when it is identified that the user is in a car through at least one of the sensor module, the GNSS module, or the communication module, attempt a second communication connection with a second external electronic device (204) around the electronic device. The processor according to an embodiment may be configured to, when a collision of the car is detected through at least one of the sensor module or the GNSS module after the second communication connection is configured, transmit a trigger signal to the first external electronic device and the second external electronic device. The processor according to an embodiment may be configured to receive information sensed by the first external electronic device and the second external electronic device from the first external electronic device and the second external electronic device, respectively, based on the trigger signal being transmitted. The processor according to an embodiment may be configured to identify accident information of the car by the collision, based on the sensed information.

The processor according to an embodiment may be configured to make a request for providing the information sensed by the second external electronic device to the second external electronic device.

The processor according to an embodiment may be configured to, when the second external electronic device does not agree to provide the sensed information, transmit information on a message to the second external electronic device to display the message asking for consent on a display of the second external electronic device.

The processor according to an embodiment may be configured to transmit the trigger signal to synchronize the electronic device with the first external electronic device and the second external electronic device, based on a time point at which the collision of the car is detected.

The processor according to an embodiment may be configured to, when it is identified that a distance between the electronic device and the second external electronic device is shorter than a predetermined distance and a change in the distance between the second external electronic device and the electronic device for a predetermined time is smaller than a predetermined value, identify the second external electronic device as an electronic device of a passenger of the car.

The processor according to an embodiment may be configured to acquire information on an acceleration, a heart rate, and an atmospheric pressure sensed by each of the first external electronic device and the second external electronic device through the sensed information.

The processor according to an embodiment may be configured to identify that the user is unconscious, based on an average value of accelerations sensed for a predetermined time being smaller than a first threshold value.

The processor according to an embodiment may be configured to, when it is identified that the user is unconscious, identify heart rates sensed by the first external electronic device or the second external electronic device and whether the heart rates increase. The processor according to an embodiment may be configured to, based on the increase in the heart rates being larger than a second threshold value, determine a state of the user or the passenger due to the collision of the car.

The processor according to an embodiment may be configured to, when it is identified that the user is unconscious, identify a first distance between the electronic device and the first external electronic device or a second distance between the electronic device and the second external electronic device. The processor according to an embodiment may be configured to, based on a change in the first distance or the second distance being larger than a third threshold value, based on a time before the collision, determine the state of the user or the passenger due to the collision of the car.

The processor according to an embodiment may be configured to, based on a change in the atmospheric pressure after the trigger signal is transmitted being larger than a fourth threshold value, determine the state of the user or the passenger due to the collision of the car.

A method of operating an electronic device 201 according to an embodiment may include an operation of configuring a first communication connection with a first external electronic device 202 worn by a user. The method of operating the electronic device according to an embodiment may include an operation of, when it is identified that the user is in a car through at least one of a sensor module 210, a GNSS module 240, or a communication module 250, attempting a second communication connection with a second external electronic device 204 around the electronic device. The method of operating the electronic device according to an embodiment may include an operation of, when a collision of the car is detected through at least one of the sensor module or the GNSS module after the second communication connection is configured, transmitting a trigger signal to the first external electronic device and the second external electronic device. The method of operating the electronic device according to an embodiment may include an operation of receiving information sensed by the first external electronic device and the second external electronic device from the first external electronic device and the second external electronic device, respectively, based on the trigger signal being transmitted. The method of operating the electronic device according to an embodiment may include an operation of identifying accident information of the car by the collision, based on the sensed information.

The method of operating the electronic device according to an embodiment may further include an operation of making a request for providing the information sensed by the second external electronic device to the second external electronic device.

The method of operating the electronic device according to an embodiment may further include an operation of, when the second external electronic device does not agree to provide the sensed information, transmitting information on a message to the second external electronic device to display the message asking for consent on a display of the second external electronic device.

The operation of transmitting the trigger signal according to an embodiment may include an operation of transmitting the trigger signal to synchronize the electronic device with the first external electronic device and the second external electronic device, based on a time point at which the collision of the car is detected.

The operation receiving the sensed information according to an embodiment may include an operation of acquiring information on an acceleration, a heart rate, and an atmospheric pressure sensed by each of the first external electronic device and the second external electronic device.

The operation of identifying the accident information according to an embodiment may include an operation of, based on an average value of accelerations sensed for a predetermined time being smaller than a first threshold value, identifying that the user is unconscious.

The operation of identifying the accident information according to an embodiment may include an operation of, when it is identified that the user is unconscious, identifying a heart rate sensed by the first external electronic device or the second eternal electronic device and whether the heart rate increases. The operation of identifying the accident information according to an embodiment may include an operation of, based on the increase in the heart rate being larger than a second threshold value, determining a state of the user or the passenger due to the collision of the car.

The operation of identifying the accident information according to an embodiment may include an operation of, when it is identified that the user is unconscious, identifying a first distance between the electronic device and the first external electronic device or a second distance between the electronic device and the second external electronic device. The operation of identifying the accident information according to an embodiment may include an operation of, based on a change in the first distance or the second distance being larger than a third threshold value, based on a time before the collision, determining the state of the user or the passenger due to the collision of the car.

The operation of identifying the accident information according to an embodiment may include an operation of, based on a change in the atmospheric pressure being larger than a fourth threshold value after the trigger signal is transmitted, determining the state of the user or the passenger due to the collision of the car.

A non-transitory computer-readable recording medium 130 storing a program according to an embodiment is provided. The program may execute an operation of configuring a first communication connection between an electronic device 201 and a first external electronic device 202 worn by a user, an operation of, when it is identified that the user is in a car through at least one of a sensor module 210, a GNSS module 240, or a communication module 250 included in the electronic device, attempting a second communication connection with a second external electronic device 204 around the electronic device, an operation of, when a collision of the car is detected through at least one of the sensor module or the GNSS module after the second communication connection is configured, transmitting a trigger signal to the first external electronic device and the second external electronic device, an operation of receiving information sensed by the first external electronic device and the second external electronic device from the first external electronic device and the second external electronic device, respectively, based on the trigger signal being transmitted, and an operation of identifying accident information of the car by the collision, based on the sensed information.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

**Claims**

1. An electronic device (201) comprising:

   a sensor module (210);
   a global navigation satellite system (GNSS) module (240);
   a communication module (250); and
   a processor (220),
   wherein the processor is configured to:

   establish, through the communication module, a first communication connection with a first external electronic device (202) worn by a user;
   when identifying, through at least one of the sensor module, the GNSS module, or the communication module, that the user is in a car, attempt a second communication connection with a second external electronic device (204) around the electronic device;
   when a collision of the car is detected through at least one of the sensor module or the GNSS module after the second communication connection is established, transmit a trigger signal to the first external electronic device and the second external electronic device;
   based on the trigger signal being transmitted, receive information sensed by the first external electronic device and the second external electronic device from the first external electronic device and the second external electronic device, respectively; and
   identify information on an accident of the car caused by the collision, based on the sensed information.

2. The electronic device of claim 1, wherein the processor is configured to transmit, to the second external electronic device, a request for providing the information sensed by the second external electronic device.

3. The electronic device of one of claims 1 and 2, wherein the processor is configured to, when the second external electronic device does not consent to provide the sensed information, transmit information on a message asking for consent to provide the sensed information to the second external electronic device so that the message is displayed on a display of the second external electronic device.

4. The electronic device of one of claims 1 to 3, wherein the processor is configured to transmit the trigger signal to synchronize the electronic device with the first external electronic device and the second external electronic device, based on a time point at which the collision of the car is detected.

5. The electronic device of one of claims 1 to 4, wherein the processor is configured to, when identifying that a distance between the electronic device and the second external electronic device is shorter than a predetermined distance and a change in the distance between the second external electronic device and the electronic device is smaller than a predetermined value for a predetermined time, identify the second external electronic device as an electronic device of a passenger in the car.

6. The electronic device of one of claims 1 to 5, wherein the processor is configured to, through the sensed information, acquire information on an acceleration, a heart rate, and an atmospheric pressure sensed by each of the first external electronic device and the second external electronic device.

7. The electronic device of one of claims 1 to 6, wherein the processor is configured to identify that the user is unconscious, based on an average value of accelerations sensed for a predetermined time being smaller than a first threshold value.

8. The electronic device of one of claims 1 to 7, wherein the processor is configured to:

   when identifying that the user is unconscious, identify a heart rate sensed by the first external electronic device or the second external electronic device and whether the heart rate is increased; and
   based on an increase in the heart rate being larger than a second threshold value, determine a state of the user or the passenger due to the collision of the car.

9. The electronic device of one of claims 1 to 8, wherein the processor is configured to:

   when identifying that the user is unconscious, identify a first distance between the electronic device and the first external electronic device or a second distance between the electronic device and the second external electronic device; and
   based on a change in the first distance or the second distance being larger than a third threshold value with reference to a time before the collision, determine the state of the user or the passenger due to the collision of the car.

10. The electronic device of one of claims 1 to 9, wherein the processor is configured to, based on a change in the atmospheric pressure being larger than a fourth threshold value after the trigger signal is transmitted, determine the state of the user or the passenger due to the collision of the car.

11. A method of operating an electronic device (201), the method comprising:

    establish a first communication connection with a first external electronic device (202) worn by a user;
    when identifying, through at least one of a sensor module (210), a GNSS module (240), or a communication module (250) included in the electronic device, that the user is in a car, attempting a second communication connection with a second external electronic device (204) around the electronic device;
when a collision of the car is detected through at least one of the sensor module or the GNSS module after the second communication connection is established, transmitting a trigger signal to the first external electronic device and the second external electronic device;
based on the trigger signal being transmitted, receiving information sensed by the first external electronic device and the second external electronic device from the first external electronic device and the second external electronic device respectively; and
identifying information on an accident of the car caused by the collision, based on the sensed information.

12. The method of claim 11, further comprising transmitting, to the second external electronic device, a request for providing the information sensed by the second external electronic device.

13. The method of one of claims 11 to 12, further comprising, when the second external electronic device does not consent to provide the sensed information, transmitting information on a message asking for consent to provide the sensed information to the second external electronic device so that the message is displayed on a display of the second external electronic device.

14. The method of one of claims 11 to 13, wherein the transmitting of the trigger signal comprises transmitting the trigger signal to synchronize the electronic device with the first external electronic device and the second external electronic device, based on a time point at which the collision of the car is detected.

15. The method of one of claims 11 to 14, wherein the receiving of the sensed information comprises acquiring information on an acceleration, a heart rate, and an atmospheric pressure sensed by each of the first external electronic device and the second external electronic device.

FIG. 1

ELECTRONIC DEVICE 101

INPUT MODULE ~150

SOUND OUTPUT MODULE ~155

DISPLAY MODULE ~160

MEMORY ~130

VOLATILE MEMORY ~132

NON-VOLATILE MEMORY ~134

INTERNAL MEMORY ~136

EXTERNAL MEMORY ~138

PROGRAM ~140

APPLICATION ~146

MIDDLEWARE ~144

OPERATING SYSTEM ~142

BATTERY ~189

PROCESSOR ~120

MAIN PROCESSOR ~121

AUXILIARY PROCESSOR ~123

POWER MANAGEMENT MODULE ~188

COMMUNICATION MODULE ~190

WIRELESS COMMUNICATION MODULE ~192

WIRED COMMUNICATION MODULE ~194

SUBSCRIBER IDENTIFICATION MODULE ~196

ANTENNA MODULE ~197

AUDIO MODULE ~170

SENSOR MODULE ~176

INTERFACE ~177

CONNECTING TERMINAL ~178

HAPTIC MODULE ~179

CAMERA MODULE ~180

SECOND NETWORK ~199

FIRST NETWORK ~198

ELECTRONIC DEVICE ~104

ELECTRONIC DEVICE ~102

SERVER ~108

~100

EP 4 620 744 A1

24

FIG. 2A

FIG. 2B

START

CONFIGURE FIRST COMMUNICATION CONNECTION WITH FIRST EXTERNAL ELECTRONIC DEVICE WORN BY USER — 301

EXECUTE ACCIDENT DETECTION APPLICATION — 303

IDENTIFY THAT USER GOT IN CAR — 305

ATTEMPT SECOND COMMUNICATION CONNECTION WITH SECOND EXTERNAL ELECTRONIC DEVICE OF ANOTHER PERSON AROUND ELECTRONIC DEVICE AND CONFIGURE SECOND COMMUNICATION CONNECTION — 307

DETECT COLLISION AND TRANSMIT TRIGGER SIGNAL — 309

RECEIVE SENSING INFORMATION FROM EACH OF FIRST EXTERNAL ELECTRONIC DEVICE AND SECOND EXTERNAL ELECTRONIC DEVICE — 311

IDENTIFY ACCIDENT INFORMATION OF CAR, BASED ON SENSING INFORMATION — 313

PROVIDE NOTIFICATION — 315

END

# FIG. 3

START

IDENTIFY SECOND EXTERNAL ELECTRONIC DEVICE OF ANOTHER PERSON AROUND ELECTRONIC DEVICE — 401

IDENTIFY DISTANCE BETWEEN ELECTRONIC DEVICE AND SECOND EXTERNAL ELECTRONIC DEVICE AND DISTANCE CHANGE — 403

405 — IDENTIFY DISTANCE < PREDETERMINED DISTANCE & DISTANCE CHANGE < PREDETERMINED VALUE? — NO

YES

IDENTIFY THAT ANOTHER PERSON CORRESPONDING TO SECOND EXTERNAL ELECTRONIC DEVICE IS PASSENGER — 407

409 — AGREE TO PROVIDE INFORMATION SENSED BY SECOND EXTERNAL ELECTRONIC DEVICE? — NO

YES

TRANSMIT MESSAGE — 411

413 — AGREE TO PROVIDE SENSED INFORMATION, BASED ON MESSAGE? — NO

YES

415 — NUMBER OF DEVICES CONNECTED TO ELECTRONIC DEVICE < PREDETERMINED NUMBER — NO

YES

CONFIGURE SECOND COMMUNICATION CONNECTION WITH SECOND EXTERNAL ELECTRONIC DEVICE — 417

NOT MAKE CONNECTION WITH SECOND EXTERNAL ELECTRONIC DEVICE — 419

END

FIG. 4

290

510

Change effective
distance

202

201

204

FIG. 5

601

(a)

(b)

FIG. 6A

Would you like to allow
another device connection and
share sensed information to — 630
provide improved SOS function?

604

Yes  No

# FIG. 6B

START

RECEIVE TRIGGER SIGNAL
FROM ELECTRONIC DEVICE ⌐701

IDENTIFY MAXIMUM IMPULSE ⌐703

FIRST VALUE < MAXIMUM IMPULSE
< SECOND VALUE? ⌐705

NO

YES

SYNCHRONIZE BASED ON TIME POINT
AT WHICH TRIGGER SIGNAL IS RECEIVED ⌐707

TRANSMIT SENSING INFORMATION
TO ELECTRONIC DEVICE ⌐709

NOT SHARE
SENSING INFORMATION ⌐711

END

FIG. 7

START

ACQUIRE SENSING INFORMATION FROM
FIRST EXTERNAL ELECTRONIC DEVICE AND
SECOND EXTERNAL ELECTRONIC DEVICE ── 801

IDENTIFY ACCELERATION VALUE
FOR PREDETERMINED TIME ── 803

ACCELERATION
VALUE < FIRST THRESHOLD VALUE? ── 805
NO

YES

IDENTIFY HEART RATE ── 807

HEART RATE
INCREASE > SECOND THRESHOLD
VALUE? ── 809
NO

YES

DETERMINE STATE OF USER OR ANOTHER
PERSON DUE TO COLLISION
OF CAR AS FIRST STATE ── 811

END

FIG. 8A

FIG. 8B

FIG. 8C

START

ACQUIRE SENSING INFORMATION FROM
FIRST EXTERNAL ELECTRONIC DEVICE AND
SECOND EXTERNAL ELECTRONIC DEVICE — 901

IDENTIFY ACCELERATION VALUE
FOR PREDETERMINED TIME — 903

ACCELERATION
VALUE < FIRST THRESHOLD
VALUE? — 905

NO

YES

IDENTIFY FIRST DISTANCE BETWEEN
ELECTRONIC DEVICE AND FIRST EXTERNAL
ELECTRONIC DEVICE AND SECOND DISTANCE
BETWEEN ELECTRONIC DEVICE AND
SECOND EXTERNAL ELECTRONIC DEVICE,
BASED ON TIME BEFORE COLLISION — 907

HEART RATE
CHANGE > THIRD THRESHOLD
VALUE? — 909

NO

YES

DETERMINE STATE OF USER OR
ANOTHER PERSON DUE TO
COLLISION OF CAR AS SECOND STATE — 911

END

FIG. 9

START

ACQUIRE SENSING INFORMATION FROM
FIRST EXTERNAL ELECTRONIC DEVICE AND
SECOND EXTERNAL ELECTRONIC DEVICE ~1001

IDENTIFY ATMOSPHERIC PRESSURE CHANGE ~1003

1005
ATMOSPHERIC
PRESSURE CHANGE > FOURTH
THRESHOLD VALUE?

NO

YES

DETERMINE STATE OF USER OR
ANOTHER PERSON DUE TO COLLISION
OF CAR AS THIRD STATE ~1007

END

FIG. 10A

FIG. 10B

FIG. 10C

(a)

(b)

FIG. 11

1201

< Ask for SOS                                              1210

Emergency contact
119

SOS emergency call
Make call to selected contact in emergency situation

Send SOS
You can send SOS by quickly pressing                           1211
home button three times

Detect car collision accident
If car collision accident situation is detected,
emergency call is made and SOS message is sent

1215    Register family device
If devices of family members are registered,
connection therebetween is allowed, sensed information is shared,
and more accurate car collision accident detection service is provided

Grandma

Wife

Son

Daughter

|||          O          <

FIG. 12

## INTERNATIONAL SEARCH REPORT

| International application No. |
|---|
| **PCT/KR2023/020381** |

| A. | CLASSIFICATION OF SUBJECT MATTER |
|---|---|

**B60R 21/013**(2006.01)i; **G04G 21/02**(2010.01)i; **B60R 21/01**(2006.01)i; **B60R 21/00**(2006.01)i

According to International Patent Classification (IPC) or to both national classification and IPC

| B. | FIELDS SEARCHED |
|---|---|

Minimum documentation searched (classification system followed by classification symbols)

B60R 21/013(2006.01); B60W 40/072(2012.01); G06F 17/30(2006.01); G06N 20/00(2019.01); G07C 5/08(2006.01); H04M 1/60(2006.01); H04M 1/725(2006.01); H04W 4/04(2009.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 차량(vehicle), 디바이스(device), 사고(accident), 감지(detection) 및 데이터(data)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | US 2018-0033220 A1 (ZENDRIVE, INC.) 01 February 2018 (2018-02-01)<br>See paragraphs [0038]-[0043], [0069]-[0072], [0090]-[0093], [0114]-[0115] and [0136]-[0168] and figures 1-7. | 1-15 |
| Y | US 2018-0077538 A1 (ZENDRIVE, INC.) 15 March 2018 (2018-03-15)<br>See paragraphs [0043] and [0052]-[0056]; claims 1 and 9; and figures 1-5. | 1-15 |
| A | US 2021-0407225 A1 (ZENDRIVE, INC.) 30 December 2021 (2021-12-30)<br>See paragraphs [0011]-[0072] and figures 1-7. | 1-15 |
| A | US 2020-0344347 A1 (GEOTOLL, INC.) 29 October 2020 (2020-10-29)<br>See paragraphs [0019]-[0048] and figures 1-10. | 1-15 |
| A | KR 10-2021-0142794 A (THINKWARE CORPORATION et al.) 26 November 2021 (2021-11-26)<br>See paragraphs [0028]-[0123] and figures 1-11. | 1-15 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 March 2024** | **22 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/020381**

| Patent document cited in search report | | | Publication date (day/month/year) | Patent family member(s) | | | Publication date (day/month/year) |
|---|---|---|---|---|---|---|---|
| US | 2018-0033220 | A1 | 01 February 2018 | US | 10137889 | B2 | 27 November 2018 |
| | | | | US | 10279804 | B2 | 07 May 2019 |
| | | | | US | 10848913 | B2 | 24 November 2020 |
| | | | | US | 11375338 | B2 | 28 June 2022 |
| | | | | US | 2017-0053461 | A1 | 23 February 2017 |
| | | | | US | 2017-0221283 | A1 | 03 August 2017 |
| | | | | US | 2017-0232963 | A1 | 17 August 2017 |
| | | | | US | 2019-0202448 | A1 | 04 July 2019 |
| | | | | US | 2021-0044928 | A1 | 11 February 2021 |
| | | | | US | 2022-0286811 | A1 | 08 September 2022 |
| | | | | US | 9818239 | B2 | 14 November 2017 |
| | | | | US | 9994218 | B2 | 12 June 2018 |
| US | 2018-0077538 | A1 | 15 March 2018 | US | 10631147 | B2 | 21 April 2020 |
| | | | | US | 11659368 | B2 | 23 May 2023 |
| | | | | US | 2018-0206090 | A1 | 19 July 2018 |
| | | | | US | 2020-0213825 | A1 | 02 July 2020 |
| | | | | US | 2023-0254673 | A1 | 10 August 2023 |
| | | | | US | 9955319 | B2 | 24 April 2018 |
| | | | | WO | 2018-049416 | A1 | 15 March 2018 |
| US | 2021-0407225 | A1 | 30 December 2021 | US | 11151813 | B2 | 19 October 2021 |
| | | | | US | 2019-0005412 | A1 | 03 January 2019 |
| US | 2020-0344347 | A1 | 29 October 2020 | US | 10708411 | B2 | 07 July 2020 |
| | | | | US | 11412080 | B2 | 09 August 2022 |
| | | | | US | 2019-0335035 | A1 | 31 October 2019 |
| KR | 10-2021-0142794 | A | 26 November 2021 | KR | 10-2022-0026566 | A | 04 March 2022 |
| | | | | KR | 10-2366489 | B1 | 25 February 2022 |
| | | | | KR | 10-2402171 | B1 | 31 May 2022 |
| | | | | US | 11790781 | B2 | 17 October 2023 |
| | | | | US | 2021-0358301 | A1 | 18 November 2021 |

Form PCT/ISA/210 (patent family annex) (July 2022)